## Europäisches Patentamt
## European Patent Office
## Office européen des brevets

(19)

(11) Veröffentlichungsnummer: **0 095 567**
**B1**

(12) # EUROPÄISCHE PATENTSCHRIFT

(45) Veröffentlichungstag der Patentschrift:
04.06.86

(51) Int. Cl.⁴: **A 61 F 2/60**

(21) Anmeldenummer: **83102933.5**

(22) Anmeldetag: **24.03.83**

(54) **Rohrmuffe zur Aufnahme und Befestigung von Rohr-Skeletteilen von künstlichen Gliedmassen.**

(30) Priorität: **21.04.82 DE 3214772**

(43) Veröffentlichungstag der Anmeldung:
**07.12.83 Patentblatt 83/49**

(45) Bekanntmachung des Hinweises auf die Patenterteilung:
**04.06.86 Patentblatt 86/23**

(84) Benannte Vertragsstaaten:
**AT BE FR GB IT NL SE**

(56) Entgegenhaltungen:
**DE - A - 2 601 906**
**DE - B - 1 073 813**
**DE - B - 1 922 619**
**DE - C - 735 068**
**US - A - 4 092 079**

**Orthopädie-Technik, Heft 5, 1980, Seiten 65-68**

(73) Patentinhaber: **Otto Bock Orthopädische Industrie Besitz- und Verwaltungs-Kommanditgesellschaft, Industriestrasse, D-3428 Duderstadt (DE)**

(72) Erfinder: **Glabiszewski, Richard, Im Puttfeld 1, D-3428 Duderstadt 15 (DE)**

(74) Vertreter: **Lins, Edgar, Dipl.-Phys. et al, Patentanwälte Gramm + Lins Theodor-Heuss-Strasse 2, D-3300 Braunschweig (DE)**

## Beschreibung

Die Erfindung betrifft eine Rohrmuffe zur Aufnahme und Befestigung von Rohr-Skeletteilen von künstlichen Gliedmassen mit einer Verstellhandhabe zur Veränderung des Innendurchmessers der Rohrmuffe.

Der Aufbau von künstlichen Gliedmassen mit Hilfe von Rohr-Skeletteilen weist eine Reihe von Vorteilen auf. Die Rohr-Skeletteile sind besonders stabil herstellbar und darüber hinaus in einfacher Form kosmetisch verkleidbar, so dass auch den kosmetischen Ansprüchen an künstlichen Gliedmassen Genüge getan werden kann.

Zur Verbindung der Rohr-Skeletteile, beispielsweise mit einem Gelenk, werden Rohrmuffen verwendet, deren zur Rohraufnahme dienendes Ende einen Längsschlitz aufweist (Orthopädie-Technik, Heft 5, 1980, S. 65–68, Abb. 3). An den Kanten des Längsschlitzes befinden sich Flanschteile, durch die eine Verstellschraube ragt, so dass die Breite des Schlitzes mittels der Verstellschraube verändert werden kann. Eine Verkleinerung der Schlitzbreite nach dem Einführen des Rohres führt zu einer Verringerung des Innendurchmessers der Rohrmuffe, so dass das Rohrteil in der Rohrmuffe festgeklemmt wird.

Eine Untersuchung der bekannten Rohrmuffen durch die Anmelderin hat ergeben, dass die bekannten Rohrmuffen trotz ihrer jahrelangen, weitverbreiteten Benutzung Nachteile aufweisen. Bei den bekannten Rohrmuffen führt eine Verringerung der Schlitzbreite mit Hilfe der Verstellschraube zu einer konischen Verkleinerung des Innendurchmessers der Rohrmuffe, wobei die Verkleinerung des Innendurchmessers am freien Ende der Rohrmuffe am grössten ist. Demzufolge wird das in die Rohrmuffe eingesetzte Rohr im wesentlichen vom äussersten Rand der Rohrmuffe gehalten. Dies führt zu einer ringförmigen, scharfkantigen Beanspruchung des eingespannten Rohres, das dadurch ringförmig und lokal eng begrenzt stark belastet wird. Diese Belastung kann bei längerem Gebrauch zu einer lokalen Schwächung des Rohres führen, so dass dieses unter Umständen keine ausreichende Stabilität mehr aufweist. Dies kann besonders bei solchen Rohrverbindungen gefährliche Folgen haben, die aufgrund ihrer Hebelverhältnisse und der auf sie wirkenden grossen Kräfte besonders stark belastet werden, so z. B. die Rohrverbindung eines Hüftgelenks mit einem Oberschenkel-Rohr. Besonders bei der Verwendung von faserverstärkten Kunststoffrohren ist eine Beschädigungsgefahr des in Belastungsrichtung orientierten Faserlaminats gegeben.

Der Erfindung liegt demgegenüber die Aufgabe zugrunde, eine Rohrmuffe der eingangs erwähnten Art zu erstellen, die in einfacher Weise eine sichere Befestigung erlaubt, die nicht zu einer gefährlichen Schwächung des eingespannten Rohres führt.

Die Aufgabe wird erfindungsgemäss gelöst durch eine mit konisch zusammenlaufenden Innenwänden versehene Auswölbung der Rohrmuffe, in der zwei mit ihren Innenflächen etwa den kreisförmigen Innenquerschnitt der Rohrmuffe fortsetzende Spannbacken an den konischen Innenwänden anliegen, deren radiale Lage durch die Verstellhandhabe veränderbar ist.

Die erfindungsgemässe Rohrmuffe sichert eine flächige Verringerung des Innenquerschnitts der Rohrmuffe mittels der Verstellhandhabe. Die Spannbacken können eine beliebig grosse axiale Länge aufweisen, so dass eine ringförmige lokale Belastung des eingespannten Rohres vermieden wird.

Besonders vorteilhaft gestaltet sich die erfindungsgemässe Rohrmuffe, wenn die Spannbacken fliegend gelagert sind. Dies kann beispielsweise mit Hilfe von Nieten geschehen, die in übergrosse Durchgangsöffnungen in den konischen Wänden der Auswölbung eingesetzt sind. Der Durchmesserunterschied zwischen den Nieten und den Durchgangsöffnungen gewährleistet die radiale Verstellbarkeit der Spannbacken.

Vorzugsweise sind die Spannbacken dabei in axialer Richtung kippbar gelagert. Hierdurch entsteht der wesentliche Vorteil, dass Rohrtoleranzen ohne weiteres gut ausgeglichen werden können. So kann auch ein aus Fertigungsgründen etwas konisch ausgefallenes Rohr flächig in die erfindungsgemässe Rohrmuffe eingespannt werden, da sich die Spannbacken aufgrund ihrer fliegenden und kippbaren Lagerung der Form des Rohres anpassen können. Die Kippbarkeit der Spannbacken in axialer Richtung kann dadurch gewährleistet werden, dass die Spannbacken nur punktförmig, also beispielsweise mit einer einzigen Niete, mit der Rohrmuffe verbunden sind. Durch die konische Ausgestaltung der Innenwände der Auswölbung, an denen die Spannbacken mit entsprechenden schrägen Flächen anliegen, ist es möglich, beide Spannbacken mit nur einer Verstellhandhabe zu verstellen, wobei die Verstellhandhabe vorzugsweise etwa in Höhe des Drehpunktes der Kippbewegung angreift.

Vorzugsweise ist die Verstellhandhabe eine Verstellschraube mit einer konischen Spitze, die in etwa halbe konische Ausnehmungen in den zueinander zeigenden Kanten der Spannbacken eingreift. Ein Anziehen der Verstellschraube drückt die Spannbacken daher nach aussen gegen die schräge Innenwand der Auswölbung, wodurch die Spannbacken auf dieser Innenwand in das Rohrinnere gleiten. Obwohl nur eine Verstellschraube vorgesehen ist, lässt sich daher eine im wesentlichen parallele Verschiebung der Spannbacken durchführen. Die Spannbacken werden daher in Umfangsrichtung nicht wesentlich gekippt, so dass auch in axialer Richtung des Rohres keine lokal begrenzte Belastung des Rohres auftritt.

Die Erfindung soll im folgenden anhand eines in der Zeichnung dargestellten Ausführungsbeispiels näher erläutert werden. Es zeigen:

Fig. 1 einen Längsschnitt durch eine Rohrmuffe mit Spannbacken;

Fig. 2 einen Querschnitt durch die Rohrmuffe aus Fig. 1 in Höhe der Verstellschraube.

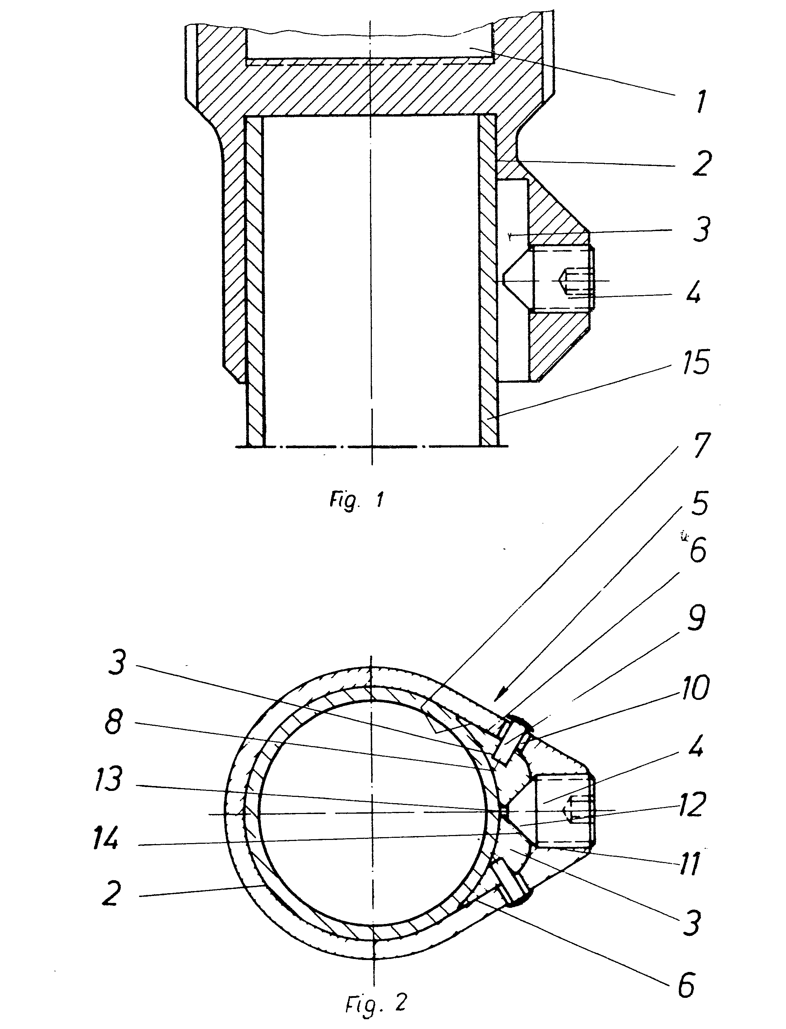

Die in Fig. 1 dargestellte Rohrmuffe weist eine nicht näher gezeigte Kammer 1 zur Befestigung an einem Gelenkteil und eine Kammer 2 zur Aufnahme eines Rohrteiles auf. Der im wesentlichen kreisförmige Querschnitt der Kammer 2 ist mit Hilfe von Spannbacken 3 veränderbar, deren radiale Lage mit Hilfe einer Verstellschraube 4 verstellbar ist. Die Spannbacken 3 erstrecken sich über nahezu die gesamte axiale Länge der Kammer 2 zur Aufnahme des Rohres.

Der Aufbau der erfindungsgemässen Rohrmuffe ist aus der Querschnittsdarstellung der Fig. 2 besonders gut erkennbar. Die Spannbacken 3 sind in einer Ausnehmung 5 der Rohrmuffe gelagert, die nach aussen konisch aufeinander zulaufende Innenwände 6 aufweist. Die Spannbacken 3 liegen mit entsprechend geneigten Flächen 7 an den konischen Innenwänden 6 der Ausnehmung 5 an. Die zur Kammer 2 zeigenden Innenwände 8 der Spannbacken 3 setzen den mit Ausnahme der Ausnehmung 5 etwa kreisförmigen Innenquerschnitt der Kammer 2 fort.

Die Spannbacken 3 sind in axialer Höhe der Verstellschraube 4, die als Inbus-Schraube ausgebildet ist, mit Hilfe eines in die zugehörige Spannbacke 3 fest eingesetzten nietartigen Bolzen 9 gehalten. Der Bolzen 9 ragt durch eine Durchgangsöffnung 10 in der konischen Wand 7 der Ausnehmung 5, wobei die Durchgangsöffnung 10 einen grösseren Durchmesser aufweist als der Schaft des Bolzens 9.

Die Verstellschraube 4 ist in der Symmetrieachse der Ausnehmung 5 in halber Höhe der Spannbacken 3 in ein Gewindeloch 11 eingeschraubt. In dem dargestellten Ausführungsbeispiel weist die Verstellschraube 4 eine kegelförmige Spitze 12 auf. Die Spannbacken 3 sind an ihren zueinander zeigenden Kanten 13 mit einer etwa halben kegelförmigen Ausnehmung 14 versehen, die jeweils mit der kegelförmigen Spitze 12 der Verstellschraube 4 zusammenwirken.

Bei gelockerter Verstellschraube 4 wird das Rohr 15 in die Kammer 2 eingeführt. Durch Anziehen der einzigen Verstellschraube 4 drückt die kegelförmige Spitze 12 der Verstellschraube 4 gegen die entsprechenden halben kegelförmigen Ausnehmungen 14 der Spannbacken 3, die dadurch auseinander gedrückt werden. Dadurch wandern die Spannbacken 3 mit ihrer schrägen Fläche 7 auf der konischen Innenwand der Ausnehmung 5 in das Innere der Kammer 2, so dass die Innenflächen 8 der Spannbacken 3 gegen das eingesetzte Rohr 15 drücken. Eventuelle Fertigungsunebenheiten des Rohres 15, wie z. B. Unrundheit oder konischer Verlauf, werden durch die fliegende Lagerung mittels des Bolzens 9 ausgeglichen, da die Spannbacken 3 in jeder Richtung, insbesondere in axialer Richtung, kippbar sind. Selbst bei Unrundheit oder konischem Verlauf der Rohrenden wird durch die Spannbacken ein über eine grosse axiale Länge erstreckter flächiger Anpressdruck auf das Rohrstück 15 ausgeübt. Eine Beschädigung und damit Schwächung des Rohres 15 durch eine ringförmige, lokal eng begrenzte Belastung kann daher bei der erfindungsgemässen Rohrmuffe nicht auftreten.

**Patentansprüche**

1. Rohrmuffe zur Aufnahme und Befestigung von Rohr-Skelettteilen von künstlichen Gliedmassen mit einer Verstellhandhabe (4) zur Veränderung des Innendurchmessers der Rohrmuffe, dadurch gekennzeichnet, dass die Rohrmuffe eine mit konisch zusammenlaufenden Innenwänden (6) versehene Auswölbung (5) aufweist, in der zwei mit ihren Innenflächen (8) etwa den kreisförmigen Innenquerschnitt der Rohrmuffe fortsetzenden Spannbacken (3), deren radiale Lage durch die Verstellhandhabe (4) veränderbar ist, an den konischen Innenwänden (6) anliegen.

2. Rohrmuffe nach Anspruch 1, dadurch gekennzeichnet, dass die Spannbacken (3) in axialer Richtung kippbar gelagert sind.

3. Rohrmuffe nach Anspruch 1 oder 2, dadurch gekennzeichnet, dass die Spannbacken (3) fliegend gelagert sind.

4. Rohrmuffe nach Anspruch 2 oder 3, dadurch gekennzeichnet, dass die Verstellhandhabe (4) etwa in Höhe des Drehpunktes der Kippbewegung angreift.

5. Rohrmuffe nach einem der Ansprüche 1 bis 4, dadurch gekennzeichnet, dass die Spannbacken (3) etwa in ihrer axialen Mitte an der Auswölbung (5) befestigt sind.

6. Rohrmuffe nach einem der Ansprüche 1 bis 5, dadurch gekennzeichnet, dass die Verstellhandhabe durch eine Verstellschraube (4) für beide Spannbacken (3) gebildet ist, die in axialer Höhe der Befestigung (9) der Spannbacken (3) an der Rohrmuffe an diesen angreift.

7. Rohrmuffe nach Anspruch 6, dadurch gekennzeichnet, dass die Verstellschraube (4) eine kegelförmige Spitze aufweist, die in etwa halbe kegelförmige Ausnehmungen (14) in den zueinander zeigenden Kanten (13) der Spannbacken (3) eingreift.

**Claims**

1. Tubular socket for receiving and fastening skeleton tubular parts of artificial limbs comprising means (4) for adjusting the inner diameter of the tubular socket, characterized in that the tubular socket comprises a recess (5) having inner walls (6) conically inclined to each other, against which two clamping grips (3) abut in said recess (5), said clamping grips (3) having inner surfaces (8) which substantially complete the circular inner section of the tubular socket and the radial position of which being adjustable by said adjusting means (4).

2. Tubular socket according to claim 1, characterized in said the clamping grips (3) are mounted pivotally in axial direction.

3. Tubular socket according to claim 1 or 2, characterized in said the clamping grips (3) are overhung.

4. Tubular socket according to claim 2 or 3, characterized in said the adjusting means (4) acts on the clamping grips (3) substantially at the height of the pivot point of the pivotal movement.

5. Tubular socket according to one of the claims 1 to 4, characterized in that the clamping grips (3) are mounted to the recess (5) substantially at their actual middle.

6. Tubular socket according to one of the claims 1 to 5, characterized in that the adjusting means is an adjusting screw (4) for both clamping grips (3) said adjusting screw (4) acting on the clamping grips (3) in the height, where the clamping grips (3) are mounted to the tubular socket.

7. Tubular socket according to claim 6, characterized in that the adjusting screw (4) has a conical tip cooperating with corresponding half conical recesses (14) being positioned in the sides (13) of the clamping grips (3) directed towards each other.

## Revendications

1. Manchon tubulaire pour la réception et la fixation de pièces tubulaires du squelette de membres artificiels, comportant un dispositif manuel de réglage (4) pour modifier le diamètre intérieur du manchon tubulaire, caractérisé en ce que le manchon tubulaire présente un renflement (5) ayant des parois intérieures (6) qui convergent avec un angle entre elles, renflement dans lequel deux mors de serrage (3), dont les surfaces intérieures (8) prolongent à peu près la section transversale intérieure circulaire du manchon tubulaire et dont la position radiale peut être modifiée au moyen du dispositif manuel de réglage (4), s'appuient contre les parois intérieures (6) formant un angle entre elles.

2. Manchon tubulaire selon la revendication 1, caractérisé en ce que les mors de serrage (3) sont supportés avec possibilité de basculement en direction axiale.

3. Manchon tubulaire selon la revendication 1 ou 2, caractérisé en ce que les mors de serrage (3) sont supportés de façon flottante.

4. Manchon tubulaire selon la revendication 2 ou 3, caractérisé en ce que le dispositif manuel de réglage (4) attaque sensiblement au niveau du point de rotation du mouvement de basculement.

5. Manchon tubulaire selon l'une des revendications 1 à 4, caractérisé en ce que les mors de serrage (3) sont fixés au renflement (5) à peu près en leur milieu axial.

6. Manchon tubulaire selon l'une de revendications 1 à 5, caractérisé en ce que le dispositif manuel de réglage est constitué d'une vis de réglage (4) pour les deux mors de serrage (3), vis qui vient en prise avec les mors de serrage (3) au niveau, par rapport à l'axe de la fixation (9) de ces mors sur le manchon tubulaire.

7. Manchon tubulaire selon la revendication 6, caractérisé en ce que la vis de réglage (4) présente une pointe conique qui vient en prise dans des évidements (14) à peu près en forme de demi-cône dans les bords (13), tournés l'un vers l'autre, des mors de serrage (3).

0095567

Fig. 1

Fig. 2